(19) Europäisches Patentamt
European Patent Office
Office européen des brevets

(11) EP 2 762 062 A1

(12) EUROPÄISCHE PATENTANMELDUNG

(43) Veröffentlichungstag:
06.08.2014 Patentblatt 2014/32

(51) Int Cl.:
A61B 5/00 (2006.01)     A61B 5/053 (2006.01)
A61B 5/08 (2006.01)

(21) Anmeldenummer: 14151452.1

(22) Anmeldetag: 16.01.2014

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(30) Priorität: 05.02.2013  DE 102013201806
10.07.2013  DE 102013213534

(71) Anmelder: Dräger Medical GmbH
23558 Lübeck (DE)

(72) Erfinder: Gärber, Yvo
23881 Breitenfelde (DE)

(74) Vertreter: UEXKÜLL & STOLBERG
Patentanwälte
Beselerstrasse 4
22607 Hamburg (DE)

(54) **Elektroimpedanztomographie-Gerät und -Verfahren**

(57) Ein EIT-Gerät mit einer Mehrzahl von Thoraxelektroden ist dazu eingerichtet, ein Elektrodenpaar als einspeisendes Elektrodenpaar mit einem Wechselstrom oder einer Wechselspannung zu versorgen, mit mehreren der übrigen Elektrodenpaare jeweils ein Spannungssignal oder Stromsignal als Messsignal aufzunehmen und sukzessive andere Elektrodenpaare aus der Mehrzahl von Elektrodenpaaren als das einspeisende Elektrodenpaare fungieren zu lassen, um aus den Messsignalen mit einem Rekonstruktionsalgorithmus eine Matrix aus Bildelementen zu rekonstruieren. Aus einer zeitlichen Abfolge der rekonstruierten Matrizen über wenigstens eine Inspiration und eine Exspiration wird für jedes Bildelement eine Zeitreihe der lokalen Impedanzänderung gewonnen. Ferner wird eine Zeitreihe der mittleren (globalen) Impedanzänderung oder eines gemessenen Beatmungsvolumens bestimmt. Für jedes Bildelement wird die zugehörige lokale Zeitreihe mit der Zeitreihe der globalen Zeitreihe bzw. des Beatmungsvolumens verglichen und ein skalarer Wert als Maß für die Abweichung berechnet. Bei Erfüllung eines Schwellenwertkriteriums wird das zugehörige Bildelement als nicht-ventiliert gekennzeichnet.

FIG. 3

EP 2 762 062 A1

**Beschreibung**

Die vorliegende Erfindung betrifft ein Elektroimpedanztomographie-Gerät mit: einer Mehrzahl von um den Thorax eines Patienten anbringbaren Elektroden, einer Steuer- und Auswerteeinheit, die durch Programmierung dazu eingerichtet ist, mindestens ein Elektrodenpaar als einspeisendes Elektrodenpaar mit einem Wechselstrom oder mit einer Wechselspannung zu versorgen, mit mehreren der übrigen Elektrodenpaare jeweils ein Spannungssignal oder Stromsignal als Messsignal aufzunehmen, sukzessive andere Elektrodenpaare als einspeisende Elektrodenpaare fungieren zu lassen, um aus den Messsignalen mit einem Rekonstruktionsalgorithmus eine Matrix aus Bildelementen zu rekonstruieren, die die Verteilung der Impedanzänderungen in der Elektrodenebene repräsentiert, und über die Zeit wiederholt Messsignale aufzunehmen und Matrizen zu rekonstruieren.

Ein derartiges Elektroimpedanztomographie-Gerät (EIT-Gerät) ist zum Beispiel aus EP 1 000 580 A1 bekannt, das zur Aufnahme eines "Elektroimpedanztomographiebildes" eines Thoraxquerschnitts eines Patienten dient.

Die elektrische Impedanztomographie ist ein Verfahren zur Rekonstruktion von Impedanzverteilungen, genauer gesagt von Impedanzänderungen bezüglich einer Referenzverteilung, in elektrisch leitfähigen Körpern. Hierzu wird an der Oberfläche des zu untersuchenden Körpers eine Vielzahl von Elektroden angebracht. In typischen Fällen wird eine ringförmige äquidistante Anordnung von 16 Elektroden verwendet, die mit einem Gurt um den Thorax eines Patienten gelegt werden können. Die Steuer- und Auswerteeinheit verfügt auch über analoge elektrische Schaltungen für die Signalverstärkung und für die Wechselstromeinspeisung und über elektronische Schaltungen zur Digitalisierung und Vorverarbeitung der Spannungssignale und über einen digitalen Signalprozessor zum Steuern des Gerätes und zur Verarbeitung der aufgenommenen Daten zur Rekonstruktion der Impedanzverteilung. Die Steuer- und Auswerteeinheit sorgt dafür, dass aufeinanderfolgend jeweils ein Paar (vorzugsweise) benachbarter Elektroden mit einem elektrischen Wechselstrom versorgt wird (z.B. 5 mA bei 50 kHz) und die elektrischen Spannungen an mehreren verbleibenden Elektrodenpaaren von der Steuer- und Auswerteeinheit erfasst werden (Grundsätzlich kann auch umgekehrt eine Wechselspannung an ein Elektrodenpaar eingespeist und die Wechselströme über mehrere verbleibende Elektrodenpaare gemessen werden); typischerweise werden die Spannungen aller verbleibenden Paare von benachbarten Elektroden erfasst, grundsätzlich ist es aber auch möglich einzelne Elektroden auszulassen, wodurch allerdings Information verloren geht. Aus der Gesamtheit aller Messsignale bei den aufeinanderfolgenden Stromeinspeisungen, bei denen das einspeisende Elektrodenpaar Schritt für Schritt um den Elektrodenring wandert, kann mit Algorithmen die Impedanzverteilung, genauer gesagt deren Änderung der Impedanz gegenüber einer Referenzverteilung, rekonstruiert werden. Die bekannten Algorithmen liefern als Rekonstruktionsergebnis eine Matrix aus 32x32 Bildelemente, wobei die Matrix für jedes Bildelement die rekonstruierte Impedanzänderung für dieses Bildelement enthält. Es wird während jedes Atemzugs eine Vielzahl von solchen Matrizen in vorgegebenen zeitlichen Abständen aufgenommen. Diese werden aufeinanderfolgend auf einer Anzeige zur Anzeige gebracht, wodurch der zeitliche Verlauf der Impedanzverteilung praktisch als Film sichtbar gemacht wird.

Die Thorax-Elektroimpedanztomographie zur Messung der regionalen Lungenventilation findet zunehmende Verbreitung in der forschungsinteressierten Intensivmedizin theoretische Modelle und experimentelle Vergleiche von EIT- mit CT-Aufnahmen des Thorax zeigen eine nahezu vollständige Proportionalität von Luftgehalt des Lungengewebes und dessen Impedanz. Die Atemzüge werden räumlich mit etwa 20% des Thoraxdurchmessers und zeitlich typischerweise mit etwa 20 bis etwa 40 Matrizen pro Sekunde aufgelöst, was ein bettseitiges Monitoring der regionalen Lungenventilation ermöglicht. Die Matrizen werden gelegentlich auch als Bilder der Impedanzverteilung (mit 32x32 = 1024 Bildelementen) oder als Frames bezeichnet.

Im Folgenden werden die Begriffe Zeitreihen von Impedanzänderungswerten und Impedanzänderungskurven mit gleicher Bedeutung verwendet, obwohl eine Zeitreihe aus diskreten Punkten im strengen Sinne keine Kurve ist. Auch in den Darstellungen werden die Zeitreihen aus Darstellungsgründen in Kurvenform als Funktionen der Zeit dargestellt.

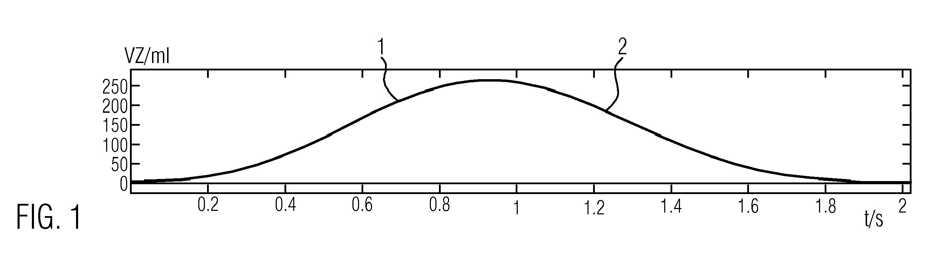

Eine mittlere Impedanzänderung für jede Matrix ergibt sich durch Integration und Normierung oder Mittelung über alle Bildelemente der Matrix. Aus der Folge der aufeinanderfolgend aufgenommenen Matrizen lässt sich so eine Zeitreihe der mittleren Impedanzänderung ableiten. Diese repräsentiert die globale Impedanzänderung im Unterschied zu der lokalen Impedanzänderung der einzelnen Bildelemente. Die mittlere Impedanzänderungszeitreihe oder Impedanzänderungskurve ist aufgrund der nahezu vollständigen Proportionalität zwischen Atemluftvolumenänderung und Impedanzänderung hochgradig mit der gemessenen Volumenkurve eines den Patienten beatmenden Beatmungsgerätes korreliert, d.h. die Impedanzänderungskurve stimmt bis auf eine Normierung fast vollständig mit der Volumenkurve der Atmung überein. Dies ist in Fig. 1 dargestellt, in der die Volumenkurve 2 des Beatmungsgerätes mit der Impedanzänderungskurve 1, die auf gleiches Integral normiert ist, verglichen ist. Über einen Atemzug ist keine Abweichung der Volumenkurve 2 von der mittleren Impedanzänderungskurve 1 erkennbar.

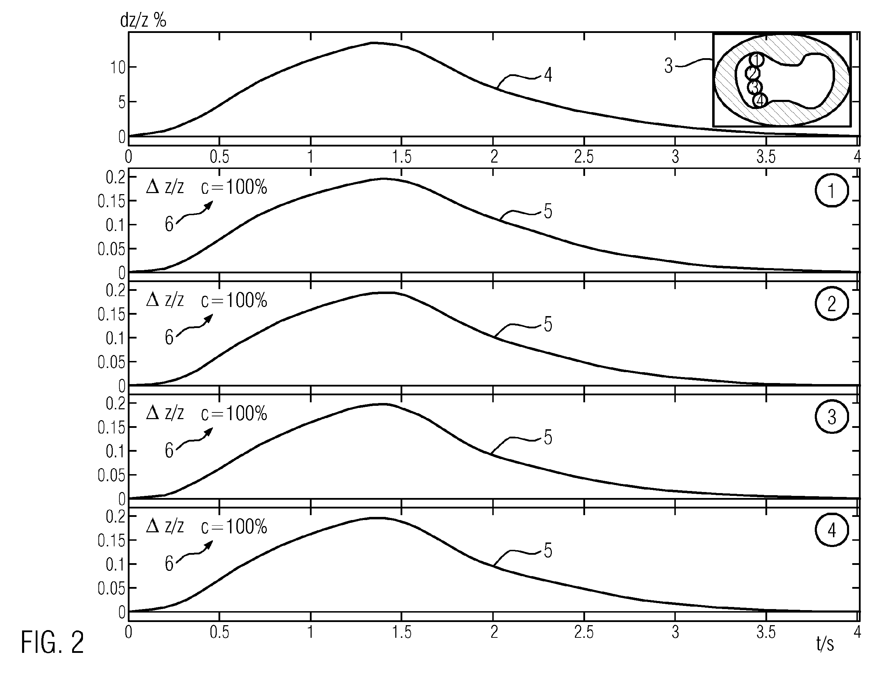

Bei räumlich und zeitlich homogener Lungenfunktion folgt daraus, dass die Korrelation der Zeitreihe der Impedanzänderungen der individuellen Bildelemente der Matrizen zur mittleren Impedanzänderungskurve und damit auch zu einer eventuell gemessenen Volumenkurve des Beatmungsgerätes sehr hoch ist. Dies ist in Fig. 2 dargestellt, worin oben die mittlere Impedanzänderungskurve über einen Atemzug dargestellt ist und darunter die Impedanzänderungskurven von vier individuellen Bildelementen 1, 2, 3 und 4, deren Lage im oberen EIT-Bild angedeutet sind. Die Beispiele aus Fig. 2 zeigen, dass die mit den Bezugszeichen 6 versehenen Korrelationen c für alle vier dargestellten Bildelemente zur mittleren Impedanzänderungskurve 100% betragen.

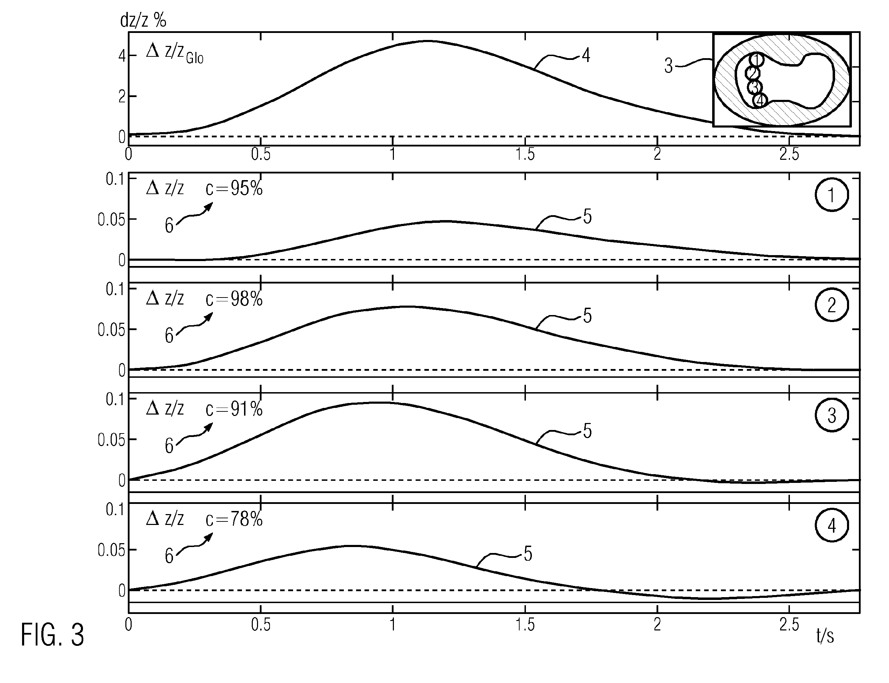

Bei zeitlich inhomogener Ventilation sind geringere Korrelationen zu erwarten, da die Luft in diesem Fall in unterschiedliche Lungengebiete unterschiedlich schnell einströmt, jedoch bleibt auch hier gültig, dass bis auf Umverteilungen mit zunehmenden Volumen die Impedanz im Wesentlichen zunimmt. In Fig. 3 ist oben wieder die mittlere Impedanzänderungskurve dargestellt, darunter sind die Impedanzänderungskurven von vier Bildelementen aus einer zeitlich stark inhomogen ventilierten COPD-Lunge (Chronicle Obstructive Pulmonary Disease) gezeigt. Die für die vier Bildelemente angegebenen Korrelationen sind hier geringer und liegen zwischen 78% und 98%.

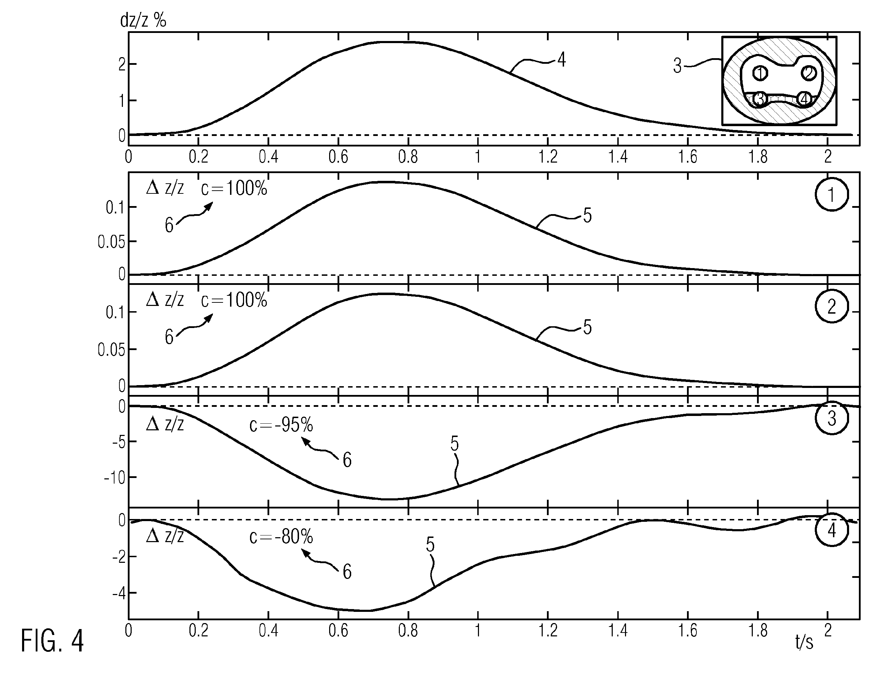

Es gibt jedoch auch Fälle, in denen das lokale zeitliche Verhalten signifikant von dem mittleren oder globalen Verhalten abweicht; im Extremfall kann die Impedanz bei zunehmenden Volumen sogar abnehmen. Dies ist in Fig. 4 gezeigt, worin oben wiederum die mittlere Impedanzänderungskurve und darunter die Impedanzänderungskurven von vier individuellen Bildelementen gezeigt ist. Die Bildelemente 3 und 4 aus dem dorsalen Lungenbereich zeigen Korrelationen von -95% und -80%, also Antikorrelationen. Ursache dafür könnte zum einen ein Rekonstruktionsartefakt sein, das als Überschwinger bezeichnet wird. Es kann sich aber auch um tatsächliche physiologische Effekte handeln, deren Ursachen noch nicht vollständig geklärt sind.

Angenommen werden hier Gewebsverschiebungen durch Zwerchfellbewegung in die Elektrodenebenen hinein, wenn die Elektroden zu tief angebracht sind, oder auch Flüssigkeitsverschiebungen bei starken Atelektasen oder Pleuralergüssen. Unabhängig von den genauen Ursachen, wäre es wünschenswert, Bildelemente deren Impedanzänderungskurven stark von der mittleren Impedanzänderungskurve abweichen als nicht-ventilierte Regionen zu erkennen und zu kennzeichnen.

Es ist Aufgabe der vorliegenden Erfindung, ein Elektroimpedanztomographie-Gerät so auszugestalten, dass Bildelemente, deren lokale zeitliche Impedanzänderung von der mittleren Impedanzänderungskurve signifikant abweicht, erkannt werden und gekennzeichnet werden können.

Zur Lösung dieser Aufgabe dient das EIT-Gerät mit den Merkmalen des Patentanspruchs 1. Vorteilhafte Ausführungsformen der Erfindung sind in den Unteransprüchen angegeben. Ein entsprechendes Verfahren ist in Anspruch 11 angegeben.

Gemäß der vorliegenden Erfindung ist vorgesehen, dass die Steuer- und Auswerteeinheit dazu eingerichtet ist, aus der zeitlichen Folge der aufgenommenen Matrizen, vorzugsweise über wenigstens eine Inspiration und eine Exspiration, für jedes Bildelement eine Zeitreihe der Impedanzänderung in diesem Bildelement zu gewinnen. Ferner wird aus der Folge der Matrizen eine Zeitreihe der mittleren Impedanzänderung (gemittelt jeweils über eine Matrix) bestimmt oder es wird eine Zeitreihe eines gemessenen Beatmungsvolumens bestimmt; die Zeitreihe des gemessenen Beatmungsvolumens wäre dann natürlich so aufgestellt, dass sie identische Zeitpunkte wie die Zeitpunkte der Aufnahme der Matrizen der Impedanzänderungen betrifft. Ferner ist die Steuer- und Auswerteeinheit dazu eingerichtet, für jedes Bildelement die zugehörige Zeitreihe der Impedanzänderung mit der Zeitreihe der mittleren Impedanzänderung oder der Zeitreihe des gemessenen Beatmungsvolumens zu vergleichen, indem für jedes Bildelement ein skalarer Wert als Maß für die Abweichung der zugehörigen Zeitreihe von der Zeitreihe der mittleren Impedanzänderung oder der Zeitreihe des gemessenen Beatmungsvolumens berechnet wird. Wenn das Maß für die Abweichung der Zeitreihe voneinander ein vorgegebenes Schwellenwertkriterium erfüllt, wird das zugehörige Bildelement durch die Steuer- und Auswerteeinheit als nicht-ventiliert bewertet und entsprechend gekennzeichnet.

Ein Vorteil der Erfindung besteht darin, dass beim Verändern von Ventilationsparametern bei künstlicher Beatmung, zum Beispiel einer PEEP-Veränderung, plötzlich ein ungewöhnliches Verhalten auftreten kann, und dass nach der Erfindung schnell sichtbar ist, wo nicht-ventilierte Bereiche vorhanden sind und gegebenenfalls wieviel Prozent der Lunge nicht ventiliert sind. Der Arzt kann dann entweder gezielt den PEEP nachjustieren, bis das ungewöhnliche Verhalten verschwindet, und/oder durch andere bildgebende Verfahren wie Computertomographie oder Magnetresonanztomographie versuchen, die morphologische Ursache für das veränderte Muster gezielt aufzufinden.

Ein anderer Vorteil der Erfindung besteht darin, dass die gekennzeichneten nicht-ventilierten Bildelemente aus weiterführenden Analysen der lokalen Ventilation wie z.B. RVD (Regional Ventilation Delay) (T. Muders et al. "Regional ventilation delay index: detection of tidal recruitment using electric impedance tomography", Vincent JL, Editor, Yearbook of intensive care and emergency medicine"), und ITV (Intratidal Variation - K. Lowhagen et al. "Regional intratidal gas distribution in acute lung injury and acute respiratory distress syndrome-assessed by electric impedance tomography", Minerva Anestesiol 76 (2010) 1024) ausgeschlossen werden können und somit die Qualität weiterführender Analysen der lokalen Ventilation verbessert werden kann. So können fehlerhafte Ergebnisse vermieden werden, die andernfalls entstehen, weil für diese nicht-ventilierten Bildelemente die Voraussetzungen für solche Analysen (Impedanzanstieg bei Luftzufuhr und umgekehrt) nicht gegeben sind.

Als Maß für die Abweichung kann für jedes Bildelement der Korrelationskoeffizient zwischen dessen Zeitreihe der Impedanzänderung und der Zeitreihe der mittleren Impedanzänderung oder der Zeitreihe des gemessenen Beatmungsvolumens berechnet werden. Als Schwellenwertkriterium wird dann geprüft, ob der Korrelationskoeffizient einen vorgegebenen Wert unterschreitet. Ein Wert des Korrelationskoeffizienten von 1 bedeutet, dass die beiden Zeitreihen keinerlei Abweichungen voneinander zeigen, ein Wert von 0 bedeutet, dass keinerlei Zusammenhang zwischen ihnen besteht, ein Wert von -1, das durch die Zeitreihen genau entgegengesetzt zueinander verhalten. Bezeichnet man zum Beispiel mit $z_k(t_i)$, $i = 1, ..., m$ die Zeitreihe der Impedanzänderung eines Bildelements $k$ und mit $z_{glo}(t_i)$, $i = 1, ..., m$ die Zeitreihe der mittleren Impedanzänderung (der Index glo steht für global), so kann der Korrelationskoeffizient für das Bildelement $k$ mit der folgenden Formel berechnet werden:

$$c_k = \frac{\frac{1}{m}\sum_{i=1}^{m}(z_k(t_i) - \overline{z}_k) \cdot (z_{glo}(t_i) - \overline{z}_{glo})}{\sqrt{\frac{1}{m}\sum_{i=1}^{m}(z_k(t_i) - \overline{z}_k)^2} \cdot \sqrt{\frac{1}{m}\sum_{i=1}^{m}(z_{glo}(t_i) - \overline{z}_{glo})^2}}$$

wobei die mit einem Querstrich versehenen Werte die Mittelwerte der Zeitreihen über die Zeitpunkte $t_1$, $i = 1, ..., m$ darstellen. Die Korrelationskoeffizienten liegen in einem Wertebereich [-1, 1]. Durch Multiplikation mit 100 erhält man die Korrelation in Prozent, die Werte im Bereich [-100%, 100%] annimmt. Im Zusammenhang mit den Ausführungsbeispielen wird meist die Korrelation angegeben.

Alternativ kann für jedes Bildelement die Kreuzkorrelationsfunktion von dessen Zeitreihe der Impedanzänderung und der Zeitreihe der mittleren Impedanzänderung oder der Zeitreihe des gemessenen Beatmungsvolumens berechnen werden. Als Maß für die Abweichung wird dann das Maximum der Kreuzkorrelationsfunktion bestimmt, und als Schwellenwertkriterium wird geprüft, ob das Maximum einen vorgegebenen Wert überschreitet.

Alternativ kann das Maß für die Abweichung berechnet werden, indem die normierte Zeitreihe der Impedanzänderung des Bildelements als Vektor aufgefasst wird und die normierte Zeitreihe der mittleren Impedanzänderung oder die normierte Zeitreihe des gemessenen Beatmungsvolumens als Vektor aufgefasst wird. Die Normierung der Zeitreihen erfolgt durch Division durch ihre Standardabweichung oder Median-Absolutabweichung, durch ihren Integralwert oder ihr Maximum oder allgemein durch eine Norm des betrachteten Vektorraumes der Zeitreihen (1-Norm, 2-Norm etc.). (Eine Norm ist allgemein eine Abbildung jedes Vektors des Vektorraumes auf eine reelle Zahl, die folgenden Bedingungen erfüllt: die Norm des Nullvektors ist 0, die Norm des Vektors $\alpha \cdot \vec{V}$ ($\alpha$ reelle Zahl) ist gleich $|\alpha|$ multipliziert mit der Norm von $\vec{V}$, und es gilt die Dreiecksungleichung). Der Betrag der Differenz der genannten Vektoren wird als Maß für die Abweichung berechnet. In diesem Fall wird als Schwellenwertkriterium geprüft, ob die Norm der Differenz einen vorgegebenen Schwellenwert überschreitet. Wenn der Differenzbetrag der Vektoren einen vorgegebenen Schwellenwert überschreitet, bedeutet dies, dass zumindest zu bestimmten Zeitpunkten der Zeitreihen diese erheblich voneinander abwichen.

In einer alternativen Ausführungsform werden wiederum die normierte Zeitreihe der Impedanzänderung des Bildelements als Vektor und die normierte Zeitreihe der mittleren Impedanzänderung oder die normierte Zeitreihe des gemessenen Beatmungsvolumens als Vektor aufgefasst und als Maß für die Übereinstimmung das Skalarprodukt der beiden genannten Vektoren gebildet. Als Schwellenwertkriterium wird dann geprüft, ob der Betrag des Skalarprodukts einen vorgegebenen Schwellenwert unterschreitet. Ist der Betrag des Skalarprodukts nämlich klein, so bedeutet dies, dass die beiden Vektoren nahezu orthogonal zueinander stehen.

In einer weiteren Ausführungsform wird das Maß für die Abweichung für jedes Bildelement berechnet, indem dessen normierte Zeitreihe der Impedanzänderung als Vektor und die normierte Zeitreihe der mittleren Impedanzänderung oder die normierte Zeitreihe des gemessenen Beatmungsvolumens als Vektor aufgefasst werden und die Norm der Summe der genannten Vektoren als Maß für die Abweichung berechnet wird, und wird als Schwellenwertkriterium geprüft, ob die Norm der Summe unterhalb eines vorgegebenen Schwellenwertes liegt. Wenn die Summe nämlich klein ist bedeutet dies, dass sich die Zeitreihen nicht konstruktiv ergänzen, sondern sich eher gegenläufig verhalten, was einer negativen Korrelation entspricht.

In einer vorteilhaften Ausführungsform kann die Steuer- und Auswerteeinheit dazu eingerichtet sein, als nicht-ventiliert bewertete Bildelemente durch einen vorgegebenen Farbton in einem EIT-Bild zur Anzeige zu bringen.

In einer weiteren Ausführungsform kann die Steuer- und Auswerteeinheit dazu eingerichtet sein, die Summe der Flächen der als nicht-ventiliert bewerteten Bildelemente zu bestimmen und diese graphisch oder alphanumerisch oder ihr Verhältnis zur gesamten Querschnittsfläche der Lunge graphisch oder alphanumerisch mit einem EIT-Bild zur Anzeige zu bringen.

Die Steuer- und Auswerteeinheit kann dazu eingerichtet sein, die Rekonstruktion der Matrizen aus den Messsignalen in Echtzeit durchzuführen und das Maß für die Abweichung für jedes Bildelement in Echtzeit zu bestimmen und das Schwellenwertkriterium in Echtzeit zu prüfen und als nicht-ventiliert bewertete Bildelemente in einem aktuellen EIT-Bild zu kennzeichnen. Alternativ kann die Steuer- und Auswerteeinheit dazu eingerichtet sein, die Messsignale zu speichern und die Rekonstruktion der Matrizen und die Bildung der Zeitreihen der Impedanzänderung der Bildelemente und der Zeitreihe der mittleren Impedanzänderung oder der Zeitreihe aus dem gespeicherten gemessenen Beatmungsvolumens in einer nachträglichen Analyse durchzuführen und für jedes Bildelement das Maß der Abweichung der Zeitreihe des Bildelements von der Zeitreihe der mittleren Impedanzänderung oder der Zeitreihe des gemessenen Beatmungsvolumens zu bestimmen und auf das Schwellenwertkriterium zu überprüfen und Bildelemente, die ein vorgegebenes Schwellenwertkriterium erfüllen, als nicht-ventiliert zu bewerten.

Die Erfindung schafft ferner ein Verfahren zur Aufnahme einer Folge von EIT-Bildern einer Querschnittsebene des Thorax eines Patienten, auf deren Umfang eine Vielzahl von Elektroden angebracht sind, wobei bei dem Verfahren mindestens ein Elektrodenpaar als einspeisendes Elektrodenpaar mit einem Wechselstrom oder einer Wechselspannung versorgt wird, mit mehreren der übrigen Elektrodenpaare jeweils ein Spannungssignal oder Stromsignal als Messsignal aufgenommen wird und sukzessive andere Elektrodenpaare aus der Mehrzahl von Elektrodenpaaren als einspeisende Elektrodenpaare betrieben werden, aus der Gesamtheit der Messsignale mit einem Rekonstruktionsalgorithmus eine Matrix aus Bildelementen rekonstruiert wird, die die Verteilung der Impedanzänderungen in der Elektrodenebene repräsentiert, über die Zeit wiederholt (vorzugsweise über wenigstens eine Inspiration und eine Exspiration) Matrizen der Impedanzänderung rekonstruiert werden, dadurch gekennzeichnet, dass aus der Folge der aufgenommenen Matrizen für jedes Bildelement eine Zeitreihe der Impedanzänderung des Bildelements gewonnen wird, eine Zeitreihe der mittleren Impedanzänderung oder eine Zeitreihe eines gemessenen Beatmungsvolumens zu gleichen Zeitpunkten wie die Zeitreihen der Bildelements bestimmt wird und für jedes Bildelement die zugehörige Zeitreihe der Impedanzänderung mit der Zeitreihe der mittleren Impedanzänderung oder der Zeitreihe des gemessenen Beatmungsvolumens verglichen wird, indem für jedes Bildelement ein skalarer Wert als Maß für die Abweichung der Zeitreihe der Impedanzänderung des Bildelements von der Zeitreihe der mittleren Impedanzänderung oder der Zeitreihe des gemessenen Beatmungsvolumens berechnet wird, und dass, wenn das Maß für die Abweichung ein vorgegebenes Schwellenwertkriterium erfüllt, das zugehörige Bildelement als nicht-ventiliert bewertet und als solches gekennzeichnet wird.

Die Erfindung wird im Folgenden anhand eines Ausführungsbeispiels im Zusammenhang mit den Figuren beschrieben, in denen:

Fig. 1 eine Beatmungsvolumenkurve und eine mittlere Impedanzänderungskurve über einen Atemzug im Vergleich zeigt,

Fig. 2 bis 4 jeweils oben eine mittlere Impedanzänderungskurve über einen Atemzug und darunter vier Impedanzänderungskurven für vier ausgewählte Bildelemente zeigen, und

Fig. 5 schematisch ein EIT-Gerät mit Elektroden und Steuer- und Auswerteeinheit zeigt.

In Fig. 1 sind die mittlere Impedanzänderungskurve 1 und eine durch das Beatmungsgerät gemessene Beatmungsvolumenkurve 2 als Funktion der Zeit über eine Inspiration und anschließende Exspiration gezeigt, wobei die beiden Kurven so gut übereinstimmen, dass sie in Fig. 1 nicht aufgelöst sind.

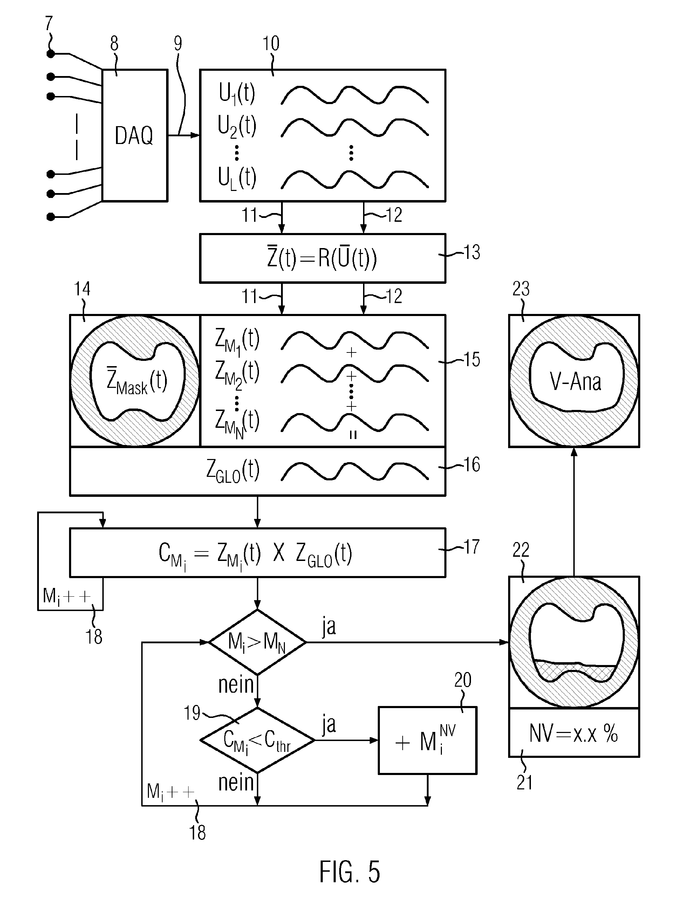

Es wird nun zunächst der Aufbau und die Funktionsweise des in Fig. 5 schematisch gezeigten EIT-Gerätes beschrieben. Das EIT-Gerät weist Elektroden 7 auf, die ringförmig um den Thorax eines Patienten anbringbar sind. Die restlichen dargestellten Komponenten sind bis auf die Anzeigeeinrichtungen 22 und 23 Teile der Steuer- und Auswerteeinheit, wobei hier verschiedene Funktionen der Steuer- und Auswerteeinheit in separaten Modulen dargestellt sind. Dies bedeutet jedoch nicht, dass diese Module physisch separate Einheiten sein müssen. Vielmehr können die verschiedenen Funktionen der Steuer- und Auswerteeinheit in einem Datenprozessor realisiert sein, wobei die in Fig. 5 dargestellten verschiedenen Module dann in verschiedenen Programmeinheiten realisiert sind.

In typischen Fällen weisen EIT-Geräte 16 Elektroden auf. Eine Datenakquisitionseinheit 8 des EIT-Gerätes sorgt für die Stromeinspeisung über ein Elektrodenpaar und die Aufnahme der Messspannungen zwischen den übrigen Paaren benachbarter Elektroden (im Falle eines 16-Elektroden-Systems gibt es unter den verbleibenden 14 Elektroden 13 Paare benachbarter Elektroden). Danach wird typischerweise über ein nächstes Elektrodenpaar Wechselstrom eingespeist und es werden typischerweise die Messspannungen aller oder einiger der übrigen Elektrodenpaare aufgenommen etc., bis jedes Elektrodenpaar einmal als einspeisendes Elektrodenpaar fungiert hat. Es ist aber in technischen Umsetzungen von EIT- Geräten auch vorstellbar, dass nicht alle der vorhandenen Elektroden zur Einspeisung von Strom oder Spannung genutzt werden, sondern einzelne Elektroden oder Elektrodenpaare bei der Einspeisung übersprungen werden. Ebenso ist es vorstellbar, dass Spannungsmessungen oder Strommessungen nicht an allen der vorhandenen Elektroden vorgenommen werden, sondern einzelne Elektroden oder Elektrodenpaare übersprungen und bei den Messungen ausgelassen werden. Für ein Gerät mit 16 Elektroden ergeben sich so für eine Aufnahme 208 Messspannungen (16 einspeisende Paare benachbarter Elektroden mit jeweils 13 Messspannungen von Paaren benachbarter Elektroden aus den verbleibenden Elektroden); diese 208 aufgenommenen Messspannungen werden auch als Frame bezeichnet. Typische EIT-Geräte arbeiten mit Frame-Raten zwischen 10 Hz und 50 Hz. Die 208 Messspannungen werden über ein Bussystem 9 zur Speichereinheit 10 des EIT-Gerätes gesendet.

Bei Verarbeitung der Daten in Echtzeit verarbeitet ein Rekonstruktionsmodul 13 die Messspannungen und rekonstruiert aus ihnen eine Matrix aus Bildelementen (typischerweise 32x32 = 1024 Bildelemente), die die örtliche Verteilung der Impedanzänderung repräsentieren. In der alternativen Datenverarbeitung zu einem späteren Zeitpunkt werden die Messspannungen gespeichert. Dies ist schematisch durch die weitere Verbindungslinie 12 angedeutet, die bedeuten soll, dass die Messspannungen zunächst gespeichert werden und zu einem späteren Zeitpunkt den weiteren Verarbeitungsschritten zugeführt werden.

In dem Zeitreihenmodul 15 für die Zeitreihen der Bildelemente werden diese summiert und normiert, um zu einer Zeitreihe der mittleren Impedanzänderung $z_{glo}$ (t) zu gelangen, die das globale Impedanzänderungsverhalten repräsentiert und in einem Modul 16 für die mittlere Impedanzänderung bereitgestellt wird.

Dabei müssen nicht sämtliche Bildelemente berücksichtigt werden, denn das Querschnittsbild eines Torsos, das in einem quadratischen Raster von Bildelementen dargestellt wird, lässt in den Ecken Bereiche zurück, die nicht zum Torso gehören und daher auch nicht in die Auswertung eingehen sollten. Dies ist in Fig. 5 durch die weißen Ecken 14 um eine schematische Darstellung eines Schnittbildes durch einen Torso angedeutet. Aber auch innerhalb des rekonstruierten Gebietes können Bildelemente vorhanden sein, die außerhalb des Lungenbereiches liegen. Bei Tierversuchen mit Schweinen liegen zum Beispiel im dorsalen Bereich starke Muskelstränge. Bei adipösen Patienten liegt am Rand eine Fettschicht. Es wird daher eine mit anderen Mitteln und Methoden erzeugte Maske verwendet, die den nicht interessierenden Außenbereich ausblendet und nur die Informationstragenden Lungenbildelemente selektiert. In Fig. 5 sind in dem Zeitreihenmodul 15 die ausgesuchten Bildelemente mit $M_1(t)$, ... $M_N(t)$ bezeichnet, die die Indizes der Zeitreihen der Impedanzänderung der ausgewerteten Bildelemente bezeichnen.

In dem Korrelationsmodul 17, 18 wird nun für jedes Bildelement $M_i$ die Zeitreihe für die Impedanzänderung $z_{Mi}(t)$ mit der Zeitreihe der mittleren Impedanzänderung $z_{glo}(t)$ verknüpft, um für jedes Bildelement einen Korrelationskoeffizienten $c_{Mi}$ zu bilden (es ist zu beachten, dass in Fig. 5 die Zeitreihen einfach als Funktionen der Zeit t bezeichnet sind, was aber nur der Vereinfachung der Darstellung dienen soll, tatsächlich sind es Zeitreihen zu diskreten Zeitpunkten gemeint). Die in dem Korrelationsmodul 17, 18 dargestellte Verknüpfung der Zeitreihen ist als Formel nur symbolisch als Verknüpfung gezeigt; die tatsächliche Ausformulierung der Verknüpfung ist zum Beispiel in der oben aufgeführten Formel für die Korrelationskoeffizienten explizit dargestellt.

Nach Bildung der Korrelationskoeffizienten in dem Korrelationsmodul 17 wird wieder eine Schleife über alle selektierten Bildelemente durchgeführt. Dies ist in Fig. 5 unten schematisch dargestellt. Es wird zunächst eine Schleife über alle Bildelemente $M_1$, ..., $M_N$ durchgeführt und abgefragt, ob das betrachtete Bildelement noch kleiner als das maximale Bildelemente $M_N$ der durch die Maske selektierten Bildelemente ist. Wenn der Index des Bildelements noch kleiner ist, wird abgefragt, ob der Korrelationskoeffizient $c_{Mi}$ kleiner als der Schwellenwert $c_{thr}$ ist. Wenn ja, wird das Bildelement $M_i$ in dem Kennzeichnungsmodul 20 als nicht-ventiliertes Bildelement gekennzeichnet. Sobald alle durch die Maske selektierten Bildelemente abgearbeitet sind, wird das EIT-Bild auf eine Anzeigeeinrichtung 22 zur Anzeige gebracht, wobei die als nicht-ventiliert bewerteten Bildelemente auf der Anzeigeeinrichtung gesondert markiert dargestellt sind. Es ist ferner ein Anzeigemodul 21 für den nicht-ventilierten Anteil vorgesehen, der den Prozentsatz der nicht-ventilierten Bildelemente angibt.

Beispiele für die Wirkungsweise der Erfindung sind in den Fig. 2 bis 4 gezeigt, in denen die Korrelation zwischen den Zeitreihen als Maß für die Abweichung gewählt ist. In den Figuren ist jeweils im oberen Graphen die mittlere Impedanzkurve (oder die Zeitreihe der gemittelten Impedanzänderung) gezeigt. In den vier Graphen darunter sind die Zeitreihen oder Kurven der Impedanzänderung für individuelle ausgewählte Bildelemente 1 bis 4 gezeigt.

Im ersten Fall in Fig. 2 handelt es sich um eine räumliche und zeitlich recht homogen ventilierte Lunge. Die Korrelationen mit der mittleren Impedanzänderungskurve 4 beträgt für alle ausgewählten Bildelemente 1, 2, 3, und 4 gerundet 100%. Alle Bildelemente innerhalb des Lungenbereichs des Querschnittsbildes werden ventiliert.

In Fig. 3 handelt es sich um eine COPD-Lunge, die aufgrund unterschiedlicher regionaler Resistivitäten und Elastizitäten zeitlich stark versetzt belüftet wird. Die dorsalen Regionen werden früher belüftet als die ventralen, aber dennoch stimmt das Muster der lokalen Bildelementkurven 5 im Wesentlichen mit der gemittelten Impedanzkurve 4 überein, so dass die Korrelationen mit knapp 80% bis 100% immer noch recht hoch sind. Die nicht abgebildeten Kreuzkorrelationskurven haben ihre Maxima alle bei 100%.

In Fig. 4 handelt es sich um einen Tierversuch mit einer durch Salzsäure künstlich geschädigten Lunge. Die Kurven oder Zeitreihen der Bildelemente im zentralen rechten und linken Lungenbereich mit den Nummern 1 und 2 sind hervorragend mit der mittleren Impedanzänderungskurve mit Korrelationen von 100% korreliert, während die Impedanzänderungskurven der Bildelemente 3 und 4 im dorsalen rechten und linken Lungenbereich kleine, aber signifikante relative Impedanzänderungen zeigen, die der Ventilation entgegenlaufen. Während sonst die Impedanz bei Luftzufuhr steigt, fällt sie dort signifikant ab. Die Korrelationen liegen infolgedessen bei -80% bis -95%. Es handelt sich hierbei nicht um Überschwinger, deren Korrelation -100% betragen würde und die dann auch an anderen Stellen auftreten würden. Es liegt der Verdacht nahe, dass dieses Verhalten mit der geschädigten Lunge im dorsalen Bereich zusammenhängt. Unabhängig von der genauen Ursache des antikorrelierenden Verhaltens findet hier keine Ventilation statt.

Die als Abweichungsmaße hier gewählten Korrelationskoeffizienten $c_{Mi}$ werden für alle durch die Maske selektierten Bildelemente mit einer Schwelle $c_{thr}$ verglichen. In diesem Beispiel wurde eine Schwelle von $c_{thr} = 0,5$ gesetzt. Lag der Korrelationskoeffizient unterhalb der Schwelle, so wurde das betroffene Bildelement als nicht-ventiliert bewertet. In dem Beispiel aus Fig. 4 betrug der Anteil der so als nicht-ventiliert bewerteten Bildelemente 6%.

In Fig. 5 ist schematisch noch ein Anzeigemodul 23 für eine weitergehende Analyse der lokalen Ventilation der Lunge dargestellt; mit dem erfindungsgemäßen EIT-Gerät und Verfahren können die als nicht-ventiliert gekennzeichneten Bildelemente in dem Analysemodul 23 von der weiteren Analyse der Lokalventilation ausgeschlossen werden, so dass sich die Qualität der weitergehenden Analyse verbessert.

**Patentansprüche**

1. Elektroimpedanztomographie-Gerät mit: einer Mehrzahl von um den Thorax eines Patienten anbringbaren Elektroden, einer Steuer- und Auswerteeinheit, die durch Programmierung dazu eingerichtet ist, mindestens ein Elektrodenpaar als einspeisendes Elektrodenpaar mit einem Wechselstrom oder mit einer Wechselspannung zu versorgen, mit mehreren der übrigen Elektrodenpaare aus der Mehrzahl von Elektroden jeweils ein Spannungssignal oder Stromsignal als Messsignal aufzunehmen und sukzessive andere Elektrodenpaare aus der Mehrzahl von Elektrodenpaaren als einspeisende Elektrodenpaare fungieren zu lassen, um aus den Messsignalen mit einem Rekonstruktionsalgorithmus eine Matrix aus Bildelementen zu rekonstruieren, die die Verteilung der Impedanzänderungen in der Elektrodenebene repräsentiert, und über die Zeit wiederholt Messsignale aufzunehmen und Matrizen zu rekonstruieren, **dadurch gekennzeichnet, dass** die Steuer- und Auswerteeinheit weiter dazu eingerichtet ist, aus der Folge der rekonstruierten Matrizen über wenigstens eine Inspiration und eine Exspiration für jedes Bildelement eine Zeitreihe der Impedanzänderung zu gewinnen, eine Zeitreihe der mittleren Impedanzänderung oder eine Zeitreihe eines gemessenen Beatmungsvolumens zu bestimmen und für jedes Bildelement die zugehörige Zeitreihe der Impedanzänderung mit der Zeitreihe der mittleren Impedanzänderung oder der Zeitreihe des gemessenen Beatmungsvolumens zu vergleichen, indem für jedes Bildelements ein skalarer Wert als Maß für die Abweichung der Zeitreihe der Impedanzänderung des Bildelements von der Zeitreihe der mittleren Impedanzänderung oder der Zeitreihe des gemessenen Beatmungsvolumens berechnet wird, und, wenn das Maß für die Abweichung ein vorgegebenes Schwellenwertkriterium erfüllt, das zugehörige Bildelement als nicht-ventiliert zu bewerten und zu kennzeichnen.

2. Elektroimpedanztomographie-Gerät nach Anspruch 1, **dadurch gekennzeichnet, dass** die Steuer- und Auswerteeinheit dazu eingerichtet ist, als Maß für die Abweichung für jedes Bildelement der Korrelationskoeffizient zwischen dessen Zeitreihe der Impedanzänderung und der Zeitreihe der mittleren Impedanzänderung oder der Zeitreihe des gemessenen Beatmungsvolumens berechnet wird und als Schwellenwertkriterium geprüft wird, ob der Korrelationskoeffizient einen vorgegebenen Wert unterschreitet.

3. Elektroimpedanztomographie-Gerät nach Anspruch 1, **dadurch gekennzeichnet, dass** die Steuer- und Auswerteeinheit dazu eingerichtet ist, für jedes Bildelement die Kreuzkorrelationsfunktion von dessen Zeitreihe der Impedanzänderung und der Zeitreihe der mittleren Impedanzänderung oder der Zeitreihe des gemessenen Beatmungsvolumens zu berechnen wird, als Maß für die Abweichung das Maximum der Kreuzkorrelationsfunktion zu bestimmen und als Schwellenwertkriterium zu prüfen, ob das Maximum einen vorgegebenen Wert unterschreitet.

4. Elektroimpedanztomographie-Gerät nach Anspruch 1, **dadurch gekennzeichnet, dass** das Maß für die Abweichung für jedes Bildelement berechnet wird, indem dessen normierte Zeitreihe der Impedanzänderung als Vektor und die normierte Zeitreihe der mittleren Impedanzänderung oder die normierte Zeitreihe des gemessenen Beatmungsvolumens als Vektor aufgefasst werden und die Norm der Differenz der genannten Vektoren als Maß für die Abweichung berechnet wird, und dass als Schwellenwertkriterium geprüft wird, ob die Norm der Differenz einen vorgegebenen Schwellenwert überschreitet, wobei die Normierung der Zeitreihen durch Division durch ihre Standardabweichung oder durch ihre Median-Absolutabweichung, durch ihren Integralwert oder durch eine Norm des betrachteten Vektorraumes erfolgt.

5. Elektroimpedanztomographie-Gerät nach Anspruch 1, **dadurch gekennzeichnet, dass** das Maß für die Abweichung für jedes Bildelement berechnet wird, indem dessen normierte Zeitreihe der Impedanzänderung als Vektor und die normierte Zeitreihe der mittleren Impedanzänderung oder die normierte Zeitreihe des gemessenen Beatmungsvolumens als Vektor aufgefasst werden und das Skalarprodukt der genannten Vektoren als Maß für die Abweichung berechnet wird, und dass als Schwellenwertkriterium geprüft wird, ob das Betrag des Skalarprodukts einen vorgegebenen Schwellenwert unterschreitet, wobei die Normierung der Zeitreihen durch Division durch ihre Standardabweichung oder durch ihre Median-Absolutabweichung, durch ihren Integralwert oder durch eine Norm des betrachteten Vektorraumes erfolgt.

6. Elektroimpedanztomographie-Gerät nach Anspruch 1, **dadurch gekennzeichnet, dass** das Maß für die Abweichung für jedes Bildelement berechnet wird, indem dessen normierte Zeitreihe der Impedanzänderung als Vektor und die normierte Zeitreihe der mittleren Impedanzänderung oder die normierte Zeitreihe des gemessenen Beatmungsvolumens als Vektor aufgefasst werden und die Norm der Summe der genannten Vektoren als Maß für die Abweichung berechnet wird, und dass als Schwellenwertkriterium geprüft wird, ob die Norm der Summe unterhalb eines vorgegebenen Schwellenwertes liegt, wobei die Normierung der Zeitreihen durch Division durch ihre Standardabweichung oder durch ihre Median-Absolutabweichung, durch ihren Integralwert oder durch eine Norm des betrachteten Vektorraumes erfolgt.

7. Elektroimpedanztomographie-Gerät nach einem der vorhergehenden Ansprüche, **dadurch gekennzeichnet, dass** die Steuer- und Auswerteeinheit dazu eingerichtet ist, als nicht-ventiliert bewertete Bildelemente durch einen vorgegebenen Farbton in einem EIT-Bild zur Anzeige zu bringen.

8. Elektroimpedanztomographie-Gerät nach einem der vorhergehenden Ansprüche, **dadurch gekennzeichnet, dass** die Steuer-und Auswerteeinheit dazu eingerichtet ist, die Summe der Flächen der als nicht-ventiliert bewerteten Bildelemente oder das Verhältnis der Summe zur Lungenquerschnittsfläche des EIT-Bildes graphisch oder alphanumerisch mit einem EIT-Bild zur Anzeige zu bringen.

9. Elektroimpedanztomographie-Gerät nach einem der vorhergehenden Ansprüche, **dadurch gekennzeichnet, dass** die Steuer- und Auswerteeinheit dazu eingerichtet ist, die Rekonstruktion der Matrizen aus den Messsignalen in Echtzeit durchzuführen und das Maß für die Abweichung für jedes Bildelement in Echtzeit zu bestimmen und das Schwellenwertkriterium in Echtzeit zu prüfen und als nicht-ventiliert bewertete Bildelemente in einem aktuellen EIT-Bild zu kennzeichnen.

10. Elektroimpedanztomographie-Gerät nach einem der Ansprüche 1 bis 8, **dadurch gekennzeichnet, dass** die Steuer- und Auswerteeinheit dazu eingerichtet ist, die Messsignale zu speichern und die Rekonstruktion der Matrizen und die Bildung der Zeitreihen der Impedanzänderung der Bildelemente und der Zeitreihe der mittleren Impedanzänderung oder der Zeitreihe aus dem gespeicherten gemessenen Beatmungsvolumens in einer nachträglichen Analyse zu vorgegebenen Zeitpunkten durchzuführen und für jedes Bildelement das Maß der Abweichung der Zeitreihe des Bildelements von der Zeitreihe der mittleren Impedanzänderung oder der Zeitreihe des gemessenen Beatmungsvolumens zu bestimmen und auf das Schwellenwertkriterium zu überprüfen und Bildelemente, die ein vorgegebenes Schwellenwertkriterium erfüllen, als nicht-ventiliert zu bewerten.

11. Verfahren zur Aufnahme einer Folge von EIT-Bildern einer Querschnittsebene des Thorax eines Patienten, auf deren Umfang eine Vielzahl von Elektroden angebracht sind, wobei bei dem Verfahren
mindestens ein Elektrodenpaar als einspeisendes Elektrodenpaar mit einem Wechselstrom oder einer Wechselspannung versorgt wird, mit mehreren der übrigen Elektrodenpaare jeweils ein Spannungssignal oder Stromsignal als Messsignal aufgenommen wird und sukzessive andere Elektrodenpaare aus der Mehrzahl von Elektrodenpaaren als einspeisende Elektrodenpaare betrieben werden,
aus der Gesamtheit der Messsignale mit einem Rekonstruktionsalgorithmus eine Matrix aus Bildelementen rekonstruiert wird, die die Verteilung der Impedanzänderungen in der Elektrodenebene repräsentiert,
über die Zeit wiederholt Matrizen der Impedanzänderung rekonstruiert werden,
**dadurch gekennzeichnet, dass**
aus der Folge der aufgenommenen Matrizen über wenigstens eine Inspiration und eine Exspiration für jedes Bildelement eine Zeitreihe der Impedanzänderung des Bildelements gewonnen wird,
eine Zeitreihe der mittleren Impedanzänderung oder eine Zeitreihe eines gemessenen Beatmungsvolumens zu gleichen Zeitpunkten wie die Zeitreihen der Bildelements bestimmt wird und
für jedes Bildelement die zugehörige Zeitreihe der Impedanzänderung mit der Zeitreihe der mittleren Impedanzänderung oder der Zeitreihe des gemessenen Beatmungsvolumens verglichen wird, indem für jedes Bildelement ein skalarer Wert als Maß für die Abweichung der Zeitreihe der Impedanzänderung des Bildelements von der Zeitreihe der mittleren Impedanzänderung oder der Zeitreihe des gemessenen Beatmungsvolumens berechnet wird, und dass, wenn das Maß für die Abweichung ein vorgegebenes Schwellenwertkriterium erfüllt, das zugehörige Bildelement als nicht-ventiliert bewertet und als solches gekennzeichnet wird.

12. Verfahren nach Anspruch 11, **dadurch gekennzeichnet, dass** die Matrizen in Echtzeit rekonstruiert, die Zeitreihen in Echtzeit gebildet werden, das Maß für die Abweichung für jedes Bildelement in Echtzeit bestimmt wird und als nicht-ventiliert bewertete Bildelemente in einer aktuellen Anzeige des EIT-Bildes kenntlich gemacht werden.

13. Verfahren nach Anspruch 11, **dadurch gekennzeichnet, dass** die Messsignale gespeichert werden und in einem nachträglichen Analyseschritt die Matrizen rekonstruiert, die Zeitreihen für die Impedanzänderungen der Bildelemente, der mittleren Impedanzänderung oder des gemessenen Beatmungsvolumens gebildet werden und zu gegebenen Zeitpunkten die Maße der Abweichungen der Bildelemente bestimmt und auf das Schwellenwertkriterium überprüft werden und Bildelemente, die ein vorgegebenes Schwellenwertkriterium erfüllen, als nicht-ventiliert bewertet werden.

FIG. 1

FIG. 2

FIG. 3

FIG. 4

EP 2 762 062 A1

13

**FIG. 5**

**Europäisches Patentamt**
**European Patent Office**
**Office européen des brevets**

# EUROPÄISCHER RECHERCHENBERICHT

**Nummer der Anmeldung**

EP 14 15 1452

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | HAHN G ET AL: "Local mechanics of the lung tissue determined by functional EIT", PHYSIOLOGICAL MEASUREMENT, INSTITUTE OF PHYSICS PUBLISHING, BRISTOL, GB, Bd. 17, Nr. 4A, 1. November 1996 (1996-11-01), Seiten A159-A166, XP020073779, ISSN: 0967-3334, DOI: 10.1088/0967-3334/17/4A/020 * Zusammenfassung * * Abbildungen 2,4,6 * * Kapitel 2.1 * * Kapitel 2.1.1 und 2.1.2 * * Kapitel 4 * ----- | 1-13 | INV. A61B5/00 A61B5/053 A61B5/08 |
| T | HAHN G ET AL: "Changes in the thoracic impedance distribution under different ventilatory conditions", PHYSIOLOGICAL MEASUREMENT, INSTITUTE OF PHYSICS PUBLISHING, BRISTOL, GB, Bd. 16, Nr. 3A, 1. August 1995 (1995-08-01), Seiten A161-A173, XP020074080, ISSN: 0967-3334, DOI: 10.1088/0967-3334/16/3A/016 * Kapitel 2.1 und 2.2 * ----- | 1,11 | RECHERCHIERTE SACHGEBIETE (IPC) A61B A61M |
| X | US 2003/216664 A1 (SUAREZ FERNANDO SIPMANN [ES]) 20. November 2003 (2003-11-20) * Abbildungen 2,8 * * Absätze [0001], [0003], [0006], [0026] - [0029], [0105] - [0108], [0142], [0147] * ----- -/-- | 1-13 | |

Der vorliegende Recherchenbericht wurde für alle Patentansprüche erstellt

| Recherchenort | Abschlußdatum der Recherche | Prüfer |
|---|---|---|
| Den Haag | 19. Mai 2014 | Albrecht, Ronald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

Europäisches
Patentamt

European
Patent Office

Office européen
des brevets

## EUROPÄISCHER RECHERCHENBERICHT

Nummer der Anmeldung

EP 14 15 1452

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG   (IPC) |
|---|---|---|---|
| X | DANIEL ROONEY ET AL: "Gravity-dependent ventilation distribution in rats measured with electrical impedance tomography; Gravity-dependent ventilation distribution in rats measured with EIT", PHYSIOLOGICAL MEASUREMENT, INSTITUTE OF PHYSICS PUBLISHING, BRISTOL, GB, Bd. 30, Nr. 10, 1. Oktober 2009 (2009-10-01), Seiten 1075-1085, XP020165116, ISSN: 0967-3334 * Abbildung 5 * * Kapitel "Electrical impedance tomography (EIT)"; Seite 1077 * * Kapitel "Filling characteristics"; Seiten 1077-107 * * "Filling characteristics"; Seite 1079 * ----- | 1-13 | |
| X | EP 2 228 009 A1 (DRAEGER MEDICAL AG [DE]) 15. September 2010 (2010-09-15) * Absätze [0013], [0014], [0018], [0021], [0023] - [0026], [0037] * ----- | 1-13 | RECHERCHIERTE SACHGEBIETE   (IPC) |

Der vorliegende Recherchenbericht wurde für alle Patentansprüche erstellt

| Recherchenort | Abschlußdatum der Recherche | Prüfer |
|---|---|---|
| Den Haag | 19. Mai 2014 | Albrecht, Ronald |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder  Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
..........................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 14 15 1452

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-05-2014

| Im Recherchenbericht angeführtes Patentdokument | Datum der Veröffentlichung | Mitglied(er) der Patentfamilie | Datum der Veröffentlichung |
|---|---|---|---|
| US 2003216664    A1 | 20-11-2003 | AT        345086 T<br>AU       8384901 A<br>AU    2001283849 B2<br>CA       2411589 A1<br>DE      60124541 T2<br>EP       1292224 A1<br>ES       2276814 T3<br>JP       4755801 B2<br>JP    2003534867 A<br>US    2003216664 A1<br>WO       0193760 A1 | 15-12-2006<br>17-12-2001<br>17-03-2005<br>13-12-2001<br>06-09-2007<br>19-03-2003<br>01-07-2007<br>24-08-2011<br>25-11-2003<br>20-11-2003<br>13-12-2001 |
| EP 2228009      A1 | 15-09-2010 | EP       2228009 A1<br>US    2010228143 A1 | 15-09-2010<br>09-09-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1000580 A1

**In der Beschreibung aufgeführte Nicht-Patentliteratur**

- Regional ventilation delay index: detection of tidal recruitment using electric impedance tomography. **T. MUDERS et al.** Yearbook of intensive care and emergency medicine

- **K. LOWHAGEN et al.** Regional intratidal gas distribution in acute lung injury and acute respiratory distress syndrome-assessed by electric impedance tomography. *Minerva Anestesiol,* 2010, vol. 76, 1024